# EUROPÄISCHE PATENTSCHRIFT

(11) **EP 0 808 174 B1**
(45) Veröffentlichungstag und Bekanntmachung des Hinweises auf die Patenterteilung: **07.05.2003**
(21) Anmeldenummer: 96901635.1
(22) Anmeldetag: 12.02.1996
(51) Int. Cl.: A61K 47/48

(54) **PARTIKULARE ARZNEIFORM**
MEDICAMENT IN PARTICLE FORM
MEDICAMENT PARTICULAIRE

(30) Priorität: 10.02.1995 AT 24895; 09.02.1996 AT 23696
(43) Veröffentlichungstag der Anmeldung: 26.11.1997
(73) Patentinhaber: Noe, Christian, 4780 Schärding (AT); Kreuter, Jörg, 61350 Bad Homburg (DE)
(72) Erfinder: NOE, Christian, A-4780 Schärding (AT); KREUTER, Jörg, D-61350 Bad Homburg (DE); ZIMMER, Andreas, D-60437 Frankfurt am Main (DE); ZOBEL, Hans-Peter, D-65439 Flörsheim (DE); STIENEKER, Frank, D-65719 Hofheim (DE); ATMACA-ABDEL AZIZ, Serap, D-64521 Gro -Gerau (DE)
(74) Vertreter: Gassner, Wolfgang, Dr.
(86) Internationale Anmeldenummer: AT9600023
(87) Internationale Veröffentlichungsnummer: WO96024377

(56) Entgegenhaltungen:
- EP-A- 0 315 219
- WO-A-94/04192
- WO-A-95/16696
- DATABASE CHEMABS CHEMICAL ABSTRACTS SERVICE, COLUMBUS, OHIO, US AN=121:103491, ELAISSARI, A. ET AL: "Adsorption of oligonucleotides onto negatively and positively charged latex particles" XP002006850 & COLLOIDS SURF., A (1994), 83(1), 25-31 CODEN: CPEAEH, 1994,
- DATABASE CHEMABS CHEMICAL ABSTRACTS SERVICE, COLUMBUS, OHIO, US AN=102:79735, SUMITOMO CHEMICAL CO., LTD., JAPAN: "Polymer beads" XP002006851 & JP,A,59 140 202 (SUMITOMO CHEM. CO.)

## Beschreibung

Die vorliegende Erfindung betrifft eine partikuläre Arzneiform, die dadurch gekennzeichnet ist, daß ein oder mehrere anionische oder partiell anionische Wirkstoffe, vorzugsweise Nucleinsäuren, Oligonucleotide, Proteine, Peptide oder biologische Makromoleküle, durch ionische Wechselwirkungen an ein Trägermaterial gebunden sind, das folgende Struktur aufweist 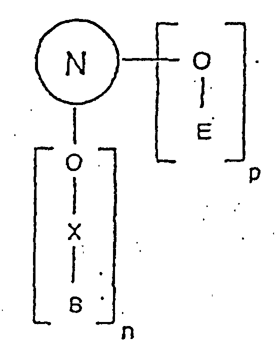 worin N = Partikelmischpolymer oder Homopolymer aus Acrylsäure oder Acrylsäureester oder Methacrylsäureestern mit einem Molverhältnis von p : n von 0 : 100 bis 99 :1; O = Sauerstoff der Estergruppe; X = verzweigte oder unverzweigte Alkylkette aus 2 - 10 Kohlenstoffen; B = -NR₂ mit R = H und Niederalkylketten in beliebiger Kombination; E = H oder Niederalkylkette bedeutet, bei einer Korngrößenverteilung von 10 bis 1000 nm. Die der Erfindung zugrundeliegenden Teilchen der Größenordnung 10 bis ca. 1000 nm haben die Zielsetzung, neben der Bindung relevanter oligomerer oder makromolekularer Strukturen, auch deren Protektion von abbauenden Enzymen und dem zielgerichteten Transport von Substanzen an den Wirkort und durch die Zellmembran zu gewährleisten.

Die der Erfindung zugrundeliegenden Partikel werden mittels Polymerisationsreaktionen aus entsprechenden Monomerbausteinen synthetisiert bzw. kondensiert. Als Monomere sind solche Substanzen geeignet, die nach der Polymerisation ein wasserunlösliches, physiologisch verträgliches Produkt liefern. Vorzugsweise verwendet man Acrylsäureester oder Methacrylsäureester mit oder ohne basische Modifikationen. Acrylsäureester oder Methacrylsäureester mit basischen Modifikationen zeichnen sich dadurch aus, daß die Alkylketten über 2 bis 10 Kohlenstoffe verfügen. Vorzugsweise sind Kettenlängen von 4 bis 10, insbesondere von 5 bis 8 Kohlenstoffe zu verwenden, da hierdurch eine optimale Verfügbarkeit der basischen Aminogruppen zur Bindung der betreffenden Substanzen gewährleistet wird.

Als Polymerisationsmechanismus kann z.B. eine radikalische oder ionische Polymerisation in einer wäßrigen oder organischen Phase oder entsprechenden Mischung aus Wasser und einem organischen Lösungsmittel wie Aceton oder Ethanol gewählt werden. Nach Beenden der Polymerisation und Entfernen des Lösungsmittels kann die Trägersubstanz in Wasser oder vorzugsweise physiologisch verträglichen Puffermedien resuspendiert werden.

Wird für die Kondensation ein Monomer mit geschützter Aminogruppe verwendet, vorzugsweise eine Trifluoracetylschutzgruppe, kann diese durch Erhitzen mit Ammoniak im Autoklaven entfernt werden. Gegebenenfalls kann ein geeigneter Reinigungsschritt, vorzugsweise Dialyse, nachgeschaltet werden.

Die so hergestellten Trägermaterialen sind partikulär und haben eine Teilchengröße von 10 bis 1000 nm, vorzugsweise ist eine Teilchengröße von 50 bis 500 nm, insbesondere von 50 bis 300 nm zu erzielen. Darüber hinaus verfügt das Trägermaterial in Wasser suspendiert über eine positive Oberflächenladung, gekennzeichnet durch ein positives Zetapotential.

Das erfindungsgemäße Trägermaterial, wird vorzugsweise zur Bindung von Nucleinsäuren wie z.B. Oligonucleotiden oder auch Peptiden aufgrund ionischer Wechselwirkungen verwendet.

Gegenüber bestehenden Techniken zur Bindung von Oligonucleotiden, z.B. Antisense-Oligonucleotiden oder Bindung von Plasmiden, die für den Gentransfer verwendet werden und die auf eine Bindung der Wirkstoffe an liposomale Träger (Lipofectin) basiert, hat die dem Patent zugrundeliegende Erfindung den wesentlichen Vorteil, daß es gegenüber liposomalen Zubereitungen ein solides, partikuläres Trägermaterial mit wesentlich höherer Stabilität in wäßrigen Suspensionen darstellt. Im Gegensatz zu Lipofectin, kann das erfindungsgemäße Trägermaterial in verschiedenen Darreichungsformen verarbeitet werden. Zum Beispiel ist die Anwendung in komprimierter Form als Implantat, welches den Wirkstoff kontrolliert über einen bestimmten Zeitraum freigibt oder eine oral anwendbare Arzneiform, die durch Komprimierung hergestellt wird, möglich.

Gegenüber bisher verwendeten partikulären Trägern wie Nanopartikeln auf Alkylcyanoacrylatbasis, hat die hier beschriebene Erfindung den Vorteil, daß spezifische funktionelle Gruppen in das Polymergerüst eingebracht werden können, welche eine gezielte Bindung des Wirkstoffes aufgrund ionischer Wechselwirkungen gewährleisten. Nach dem Stand der Technik zeigen bisher entwickelte partikuläre Träger, sowohl auf Alkylcyanoacrylatbasis als auch auf Methacrylatbasis keine oder nur eine unzureichende Bindung. Die bisher bekannten nanopartikulären Arzneiformen verfügen jedoch nicht über die in der vorliegenden Erfindung beschriebenen basischen funktionellen Gruppen. Eine Bindung von Wirkstoffen an bisher bekannte Nanopartikel, zumeist Methylmethacrylatnanopartikel, findet unspezifisch durch Adsorptionseffekte von lipophilen Substanzen an Oberflächen oder durch Einkapselung bzw. Einbettung in das Polymergerüst statt.

Ferner sind Partikel auf Acrylatbasis in mikropartikulärer Form bekannt, die über basische Modifikationen verfügen. Diese Trägermaterialien unterscheiden sich wesentlich von der vorliegenden Erfindung dadurch, daß sie über eine Korngröße > 1 µm verfügen. Solche Zubereitungen werden häufig als Ausgangsprodukte zur Herstellung von Überzügen auf Tabletten oder zur Herstellung von Depotmatrixsystemen für perorale oder transdermale Arzneiformen verwendet (EP 0 164 669 A2, EP A 0 315 219). Eine parenterale Applikation in Form einer i.v. Applikation solcher mikropartikulärer Trägermaterialien als eigenständige Arzneiform ist bisher nicht bekannt.

### Beispiel 1: Herstellung eines Trägerpolymers mit 50 mol% basischem Anteil

Eine Monomerlösung, bestehend aus 1% Methylmethacrylat (MMA) und 1% Trifluoracetylaminohexylmethacrylat Monomer in 44% Aceton, werden bei 63°C nach Polymerisationsstart durch eine 0,03% Ammoniumperoxodisulfatlösung (APS), unter Rühren (400 U/min) polymerisiert.

Im Detail wird Aqua dem. 1 h unter Einleiten von Stickstoff ausgekocht. 40 g davon und 33 ml Aceton (26,1 g) werden in ein 100 ml Schraubdeckelgefäß gegeben. Bei einer Temperatur von 55°C werden zunächst Trifluoracetylaminohexylmethacrylat Monomer/Aceton-Lösung (1:1), MMA und danach 450 µl APS-Starterlösung (5%ig), zugefügt. Anschließend wird auf 63°C aufgeheizt und bei dieser Temperatur 20 Stunden gerührt. Danach werden die Schraubdeckel der Gefäße entfernt und noch 1,5 h bei dieser Temperatur weitergerührt. Zum Schluß wird mit Aqua dem. ad 50 g ergänzt.

Die entstandene Dispersion wird im Verhältnis 1:2 (g/g) mit Ammoniak konz. versetzt und 60 min lang bei 121°C autoklaviert. Danach wird 3 Tage in fließendem Wasser und 1 Tag in Aqua dem. dialysiert.

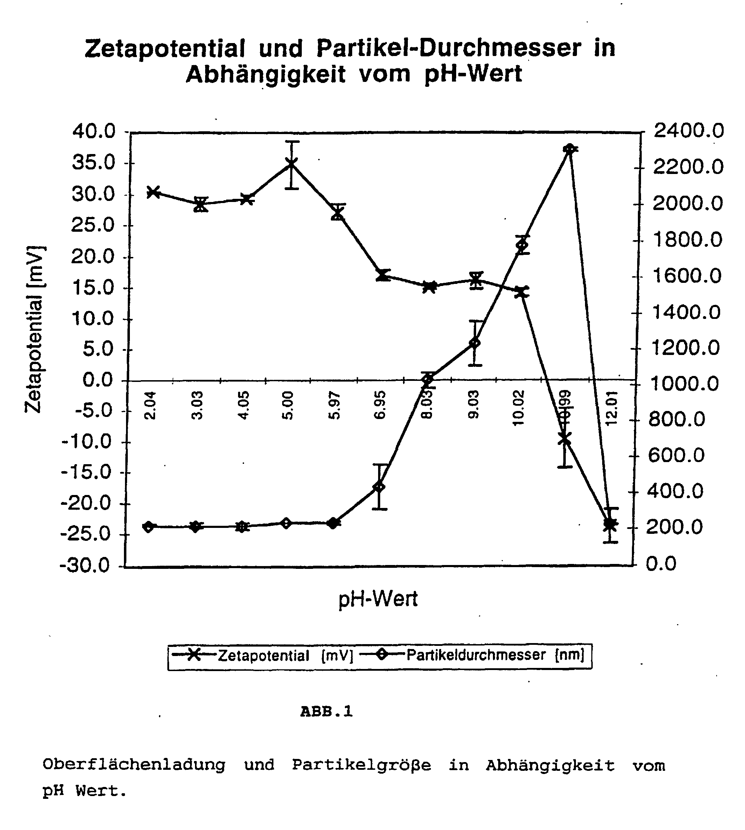

Das erhaltene Polymer wird abschließend gefriergetrocknet. Partikelgröße und Oberflächenladung sind aufgrund des Gehaltes an basischen Gruppen vom pH abhängig. Durch Bildung von Aggregaten können auch Teilchengrößen oberhalb des aus der Polymerisation stammenden Größenbereiches von 10 bis 1000 nm resultieren (Abb. 1).

### Beispiel 2: Bindung von Oligonucleotiden

7,5 mg des nach Beispiel 1 hergestellten Trägerpolymers werden in 1,0 ml destilliertem Wasser unter Zuhilfenahme von Ultraschall dispergiert. Es resultiert daraus eine 0,75%ige Trägersuspension von der 60 µl bei Raumtemperatur (20°C) mit 30 µl Phosphatpuffer (70mM) pH 7,0 versetzt werden. Diese Trägersuspension wird mit 60 µl Oligonucleotidlösung (36,4 µg/ml) gemischt und bei 20°C 2 Stunden inkubiert.

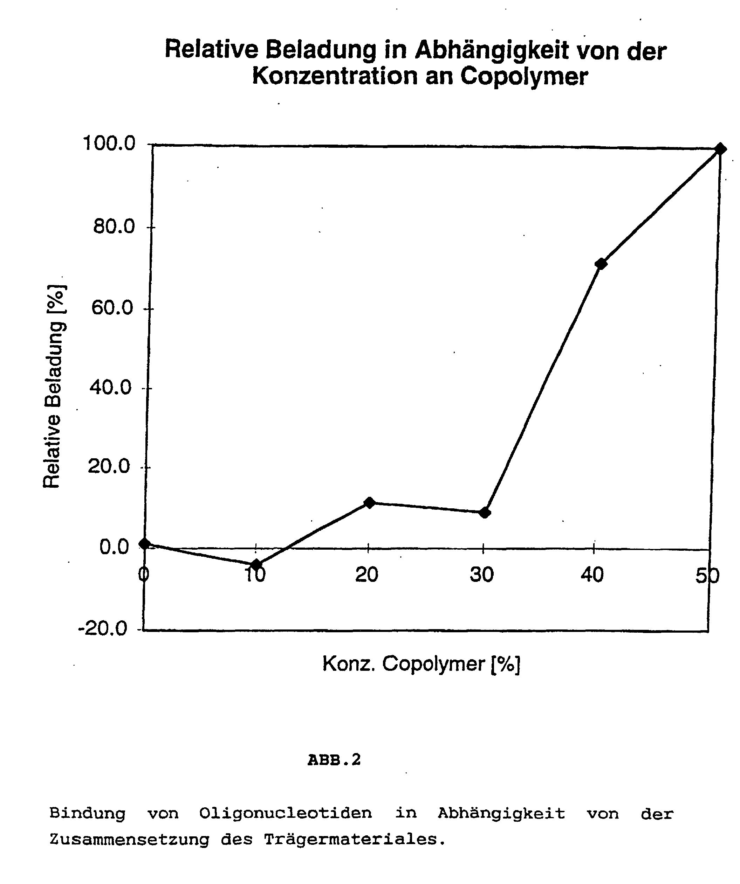

Zur Bestimmung des gebundenen Oligonucleotidanteiles, werden 100 µl dieser Partikelsuspension in einer Ultrazentrifuge bei ca. 100.000 g in 30 Minuten abzentrifugiert. Der nicht gebundene Anteil wird photometrisch im Überstand bestimmt (Abb. 2).

### Beispiel 3: Bindung von Peptiden gezeigt am Beispiel TGF α

20 mg des nach Beispiel 1 hergestellten Trägerpolymers werden in 1,0 ml destilliertem Wasser unter Zuhilfenahme von Ultraschall dispergiert. Es resultiert daraus eine 2%ige Trägersuspension von der 125 µl mit 62,5 µl einer 0,3% BSA haltigen PBS-Pufferlösung, pH 7,4 versetzt werden. Dieser Dispersion werden 62,5 µl einer TGF α Lösung (30 µg/ml) zugemischt und bei Raumtemperatur 24 Stunden inkubiert.

Die Bestimmung des gebundenen Peptidanteiles erfolgt analog dem Beispiel 2. TGF α kann mittels eines geeigneten ELISA-Tests oder durch Messung der an dem Partikel verbleibenden Radioaktivität nach Markierung mit ¹²⁵I bestimmt werden.

Es werden durchschnittlich 70-90% der eingesetzten Peptidmenge an den partikulären Träger gebunden.

### Beispiel 4: Herstellung eines Trägerpolymers mit 30% basischem Anteil

Eine Monomerlösung, bestehend aus 2,1% Methylmethacrylat (MMA) und 0,9% Trimethylaminoethylmethacrylat-Monomer in 10% Aceton, wird bei 78°C nach Polymerisationsstart durch eine 0,03% Ammoniumperoxodisulfatlösung (APS), unter Rühren (400 U/min) polymerisiert.

Im Detail wird Aqua dem. 1 h unter Einleiten von Stickstoff ausgekocht. 67,5 g davon und 7,5 ml Aceton werden in ein 100 ml Schraubdeckelgefäß gegeben. Bei einer Temperatur von 78°C werden die Monomere und anschließend die 450 µl APS-Starterlösung (5%ig) zugefügt. Anschließend wird 20 Stunden gerührt. Danach werden die Schraubdeckel der Gefäße entfernt und noch 1,5 h bei dieser Temperatur weitergerührt. Zum Schluß wird mit Aqua dem. ad 75 g ergänzt.

Weiterhin wird 3 Tage in fließendem Wasser und 1 Tag in Aqua dem. dialysiert und anschließend das erhaltene Polymer gefriergetrocknet.

### Beispiel 5: Bindung von Oligonucleotiden

Eine Dispersion der Konzentration 200 µg/ml des nach Beispiel 4 hergestellten Trägerpolymers in PBS Puffer pH 7,4, wird mit steigenden Konzentrationen eines Oligonucleotids (10-100 µg/ml in PBS) für 3 h bei Raumtemperatur (20°C) inkubiert.

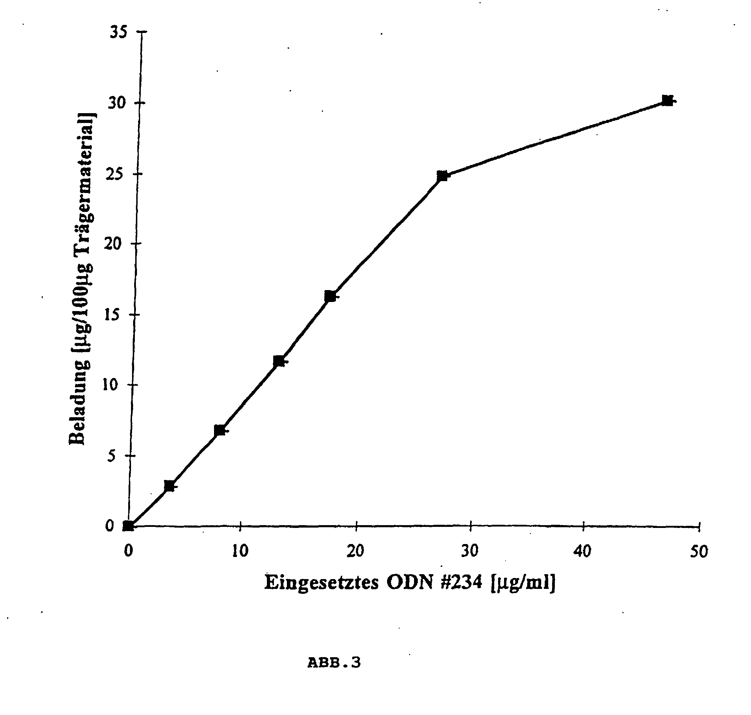

Zur Bestimmung des gebundenen Oligonucleotidanteiles, werden die Partikelsuspensionen in einer Ultrazentrifuge bei ca. 100.000 g 30 min zentrifugiert. Der nicht gebundene Anteil Oligonucleotid wird HPLC-analytisch im Überstand bestimmt (Abb. 3).

## Patentansprüche

1. Partikuläre Arzneiform, **dadurch gekennzeichnet, daß** ein oder mehrere anionische oder partiell anionische Wirkstoffe, vorzugsweise Nucleinsäuren, Oligonucleotide, Proteine, Peptide oder biologische Makromoleküle, durch ionische Wechselwirkungen an ein Trägermaterial gebunden sind, das folgende Struktur aufweist 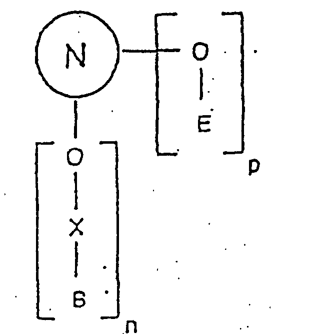 worin N = Partikelmischpolymer oder Homopolymer aus Acrylsäure oder Acrylsäureester oder Methacrylsäureestern mit einem Molverhältnis von p : n von 0 : 100 bis 99 : 1; O = Sauerstoff der Estergruppe; X = verzweigte oder unverzweigte Alkylkette aus 2 - 10 Kohlenstoffen; B = -NR₂ mit R = H und Niederalkylketten in beliebiger Kombination; E = H oder Niederalkylkette bedeutet, bei einer Korngrößenverteilung von 10 bis 1000 nm.

2. Partikuläre Arzneiform nach Anspruch 1, **dadurch gekennzeichnet, daß** X verzweigte oder unverzweigte Alkylkette aus 4-10 Kohlenstoffatomen darstellt.

3. Partikuläre Arzneiform nach Anspruch 2, **dadurch gekennzeichnet, daß** X eine lineare Alkylkette mit 5 bis 8 Kohlenstoffen darstellt.

4. Partikuläre Arzneiform nach Anspruch 1, **dadurch gekennzeichnet, daß** X verzweigte oder unverzweigte Alkylkette aus 2 oder 3 Kohlenstoffatomen darstellt, bei einer Korngrößenverteilung von 10 bis 500 nm.

5. Partikuläre Arzneiform nach Anspruch 4, **dadurch gekennzeichnet, daß** ein oder mehrere peptidische Substanzen an das Trägermaterial gebunden sind.

6. Partikuläre Arzneiform nach Anspruch 4, **dadurch gekennzeichnet, daß** Proteine oder andere biologisch aktive Makromoleküle an das Trägermaterial gebunden sind.

7. Partikuläre Arzneiform nach einem oder mehreren der Ansprüche 1 bis 6, **dadurch gekennzeichnet, daß** B eine Ammoniumgruppe bedeutet und demgemäß als -N⁺R₃ vorliegt, wobei R die in Anspruch 1 angegebene Bedeutung hat.

8. Partikuläre Arzneiform nach einem oder mehreren der Ansprüche 1 bis 7, **dadurch gekennzeichnet, daß** der anionische oder partiell anionische Wirkstoff eine Nucleinsäure, ein Oligonucleotid oder ein Peptid ist.

9. Verwendung der partikulären Trägermaterialien nach einem oder mehreren der Ansprüche 1 bis 7 als Träger für Arzneistoffe.

10. Verwendung der partikulären Trägermaterialien nach einem oder mehreren der Ansprüche 1 bis 7 als Träger für Nucleinsäuren und unmodifizierte oder modifizierte Oligonucleotide.

11. Verwendung der partikulären Trägermaterialien nach einem oder mehreren der Ansprüche 1 bis 7 als Träger für Peptide bzw. peptidische Substanzen.

12. Verwendung der partikulären Trägermaterialien nach einem oder mehreren der Ansprüche 1 bis 7 als Träger für Peptide bzw. Proteine oder andere biologisch aktive Makromoleküle.

## Claims

1. A medicament in particulate form, **characterised in** a carrier material and one or more anionic or partially anionic active ingredients, preferably nucleic acids, oligonucleotids, proteins, peptides or biological macromolecules, which are bound to said carrier material by ionic interactions, said carrier material having the following structure: 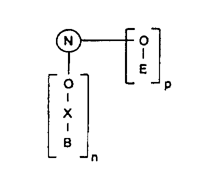 wherein N = particulate mixed polymer or homopolymer of acrylic acid or acrylate or methacrylates in a molar ratio of p:n of 0:100 to 99:1; O = oxygen of the ester group; X = branched or unbranched alkyl chain with 2 to 10 carbon atoms; B = -NR₂ where R = H and low alkyl chains in arbitrary combination; E = H or low alkyl chain, with a particle size distribution of 10 to 1000 nm.

2. A medicament according to claim 1, wherein X is a branched or unbranched alkyl chain with 4 to 10 carbon atoms.

3. A medicament according to claim 2, wherein X is a linear alkyl chain with 5 to 8 carbon atoms.

4. A medicament according to claim 1, wherein X is a branched or unbranched alkyl chain with 2 or 3 carbon atoms, with a particle size distribution of 10 to 500 nm.

5. A medicament according to claim 4, wherein one or more peptide substances are bound to said carrier material.

6. A medicament according to claim 4, wherein proteins or other biologically active macromolecules are bound to said carrier material.

7. A medicament according one or several of claims 1 to 6, wherein B represents an ammonium group and is present in the form -N⁺R₃, where R has the meaning specified in claim 1.

8. A medicament according to one or several of claims 1 to 7, wherein said anionic or partially ionic active ingredient is a nucleic acid, an oligonucleotide, or a peptide.

9. A use of the carrier materials in particulate form according to one or several of claims 1 to 7 as a carrier for pharmaceuticals.

10. A use of the carrier materials in particulate form according to one or several of claims 1 to 7 as a carrier for nucleic acids and unmodified or modified oligonucleotids.

11. A use of the carrier materials in particulate form according to one or several of claims 1 to 7 as a carrier for peptides and peptide substances.

12. A use of the carrier materials in particulate form according to one or several of claims 1 to 7 as a carrier for peptides, and proteins, respectively, and other biologically active macromolecules.

## Revendications

1. Médicament particulaire, **caractérisé en ce qu'**un ou plusieurs principes actifs anioniques ou partiellement anioniques, de préférence des acides nucléiques, des oligonucléotides, des protéines, des peptides ou des macromolécules biologiques, sont liés par des interactions ioniques à un matériau de support, qui présente la structure suivante 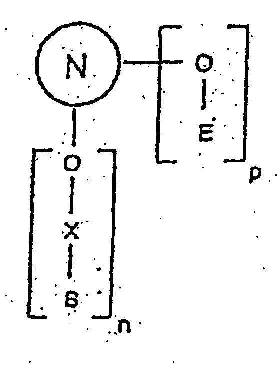 dans laquelle N = copolymère particulaire ou homopolymère d'acide acrylique ou d'ester d'acide acrylique ou d'esters d'acide méthacrylique présentant un rapport molaire p:n compris entre 0:100 et 99:1 ; O = oxygène du groupe ester ; X = chaîne alkyle ramifiée ou non ramifiée comportant 2 à 10 atomes de carbone ; B = -NR₂, avec R = H et des chaînes alkyle inférieur selon une combinaison quelconque: E = H ou une chaîne alkyle inférieur, avec une répartition granulométrique comprise entre 10 et 1 000 nm.

2. Médicament particulaire selon la revendication 1, **caractérisé en ce que** X représente une chaîne alkyle ramifiée ou non ramifiée comportant 4 à 10 atomes de carbone.

3. Médicament particulaire selon la revendication 2, **caractérisé en ce que** X représente une chaire alkyle linéaire comportant 5 à 8 atomes de carbone.

4. Médicament particulaire selon la revendication 1, **caractérisé en ce que** X représente une chaîne alkyle ramifiée ou non ramifiée comportant 2 à 3 atomes de carbone, avec une répartition granulométrique comprise entre 10 et 500 nm.

5. Médicament particulaire selon la revendication 4, **caractérisé en ce qu'**une ou plusieurs substances peptidiques sont liées au matériau de support.

6. Médicament particulaire selon la revendication 4, **caractérisé en ce que** des protéines ou d'autres macromolécules biologiquement actives sont liées au matériau de support.

7. Médicament particulaire selon une ou plusieurs des revendications 1 à 6, **caractérisé en ce que** B représente un groupe ammonium et se présente par conséquent sous forme de -N⁺R₃, R ayant la signification mentionnée dans la revendication 1.

8. Médicament particulaire selon une ou plusieurs des revendications 1 à 7, **caractérisé en ce que** le principe actif anionique ou partiellement anionique est un acide nucléique, un oligonucléotide ou un peptide.

9. Utilisation des matériaux de support particulaires selon une ou plusieurs des revendications 1 à 7 en tant que supports de médicaments.

10. Utilisation des matériaux de support particulaires selon une ou plusieurs des revendications 1 à 7 en tant que supports d'acides nucléiques et d'oligonucléotides non modifiés ou modifiés.

11. Utilisation des matériaux de support particulaires selon une ou plusieurs des revendications 1 à 7 en tant que supports de peptides ou de substances peptidiques.

12. Utilisation des matériaux de support particulaires selon une ou plusieurs des revendications 1 à 7 en tant que supports de peptides ou de protéines, ou d'autres macromolécules biologiquement actives.
